(19) Europäisches Patentamt / European Patent Office / Office européen des brevets

(11) EP 3 434 792 A1

# (12) EUROPEAN PATENT APPLICATION

(43) Date of publication:
**30.01.2019 Bulletin 2019/05**

(21) Application number: **18185116.3**

(22) Date of filing: **24.07.2018**

(51) Int Cl.:
***C12R 1/07*** *(2006.01)* ***C12N 9/14*** *(2006.01)*
***B01D 53/70*** *(2006.01)* ***C02F 3/34*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.07.2017 KR 20170093684**

(71) Applicant: **Samsung Electronics Co., Ltd.**
**Gyeonggi-do 16677 (KR)**

(72) Inventors:
- **JUNG, Yukyung**
**16678 Gyeonggi-do (KR)**
- **BYUN, Jongwon**
**16678 Gyeonggi-do (KR)**
- **KIM, Taeyong**
**16678 Gyeonggi-do (KR)**
- **PARK, Jinhwan**
**16678 Gyeonggi-do (KR)**
- **SONG, Seunghoon**
**16678 Gyeonggi-do (KR)**

(74) Representative: **Grünecker Patent- und Rechtsanwälte**
**PartG mbB**
**Leopoldstraße 4**
**80802 München (DE)**

## (54) BACILLUS SAITENS DECOMPOSING FLUORINE-CONTAINING COMPOUND, MICROORGANISM INCLUDING GENE DERIVED FROM BACILLUS SAITENS, AND METHOD OF REDUCING CONCENTRATION OF FLUORINE-CONTAINING COMPOUND IN SAMPLE BY USING BACILLUS SAITENS

(57) Provided are a microorganism having activity in reducing a concentration of a fluorine-containing compound in a sample, a microorganism including a gene derived from the microorganism, and a method of reducing the concentration of the fluorine-containing compound in the sample by using the microorganism or recombinant microorganism.

FIG. 3

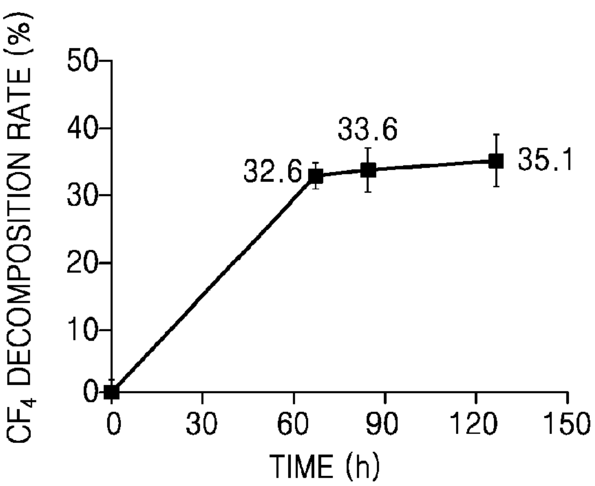


EP 3 434 792 A1

```
<110>    Samsung Electronics Co., Ltd.

<120>    Bacillus saitens decomposing fluorine-containing compound,
         microorganism including gene derived from bacillus saitens,
         and method of reducing concentration of fluorine-containing
         compound in sample by using bacillus saitens

<130>    EP118419FZTR

<140>    not yet assigned
<141>    herewith

<150>    10-2017-0093684
<151>    2017-07-24

<160>    12

<170>    KopatentIn 2.0

<210>    1
<211>    230
<212>    PRT
<213>    Artificial Sequence

<220>
<223>    Bacillus saitens KCTC 13219BP


<400>    1
Met Gly Tyr Lys Ala Met Leu Phe Asp Leu Asp Asp Thr Leu Leu Asp
  1               5                  10                  15

Arg Asp Lys Ala Val Glu Ala Leu Phe Leu Ile Val Leu Glu Lys Cys
             20                  25                  30

Tyr Glu Asn Val Asp Gly Ala Ala Lys Ser Asn Met Leu Gln Lys Phe
         35                  40                  45

Lys Glu Tyr Asp Lys Arg Glu Tyr Gly Ile Ser Asn Lys Thr Thr Val
     50                  55                  60

Leu Glu Ser Leu Phe Asp Glu Phe Thr Pro Arg Tyr Arg Leu Pro Arg
 65                  70                  75                  80

Asn Tyr Ile Gln Asp Phe Trp Asn Asn Asn Phe Pro Arg Cys Phe Ser
                 85                  90                  95

Ile Asp Gln Asn Thr Ile His Phe Leu Asn Gln Ile Lys Lys His Cys
            100                 105                 110

Lys Val Gly Ile Ile Thr Asn Gly Ser Thr Gln Arg Gln Lys Ala Lys
        115                 120                 125

Ile Phe Asn Thr Asn Leu Asn Lys Tyr Phe Glu Thr Ile Ile Ile Ser
    130                 135                 140

Glu Glu Val Gly Phe Ser Lys Pro Asp Lys Arg Ile Phe Glu Leu Ala
145                 150                 155                 160

Leu Asn Lys Leu Asn Leu Gln Pro Glu Asn Thr Leu Phe Val Gly Asp
                165                 170                 175

Asp Leu Glu Lys Asp Ile Ala Gly Pro Gln Asn Ala Asn Ile Lys Gly
```

```
            180                 185                 190

Val Trp Phe Asn Pro Gln Lys Ile Lys Asn Thr Thr Lys Ile Gln Pro
        195                 200                 205

Tyr Ala Glu Ile Asn Thr Leu Asp Ser Leu Leu Ser Tyr Val Thr Pro
    210                 215                 220

Gln Tyr Phe Tyr Asn Lys
225                 230


<210>   2
<211>   693
<212>   DNA
<213>   Artificial Sequence

<220>
<223>   Bacillus saitens KCTC 13219BP


<400>   2
atgggttata aagcgatgct gtttgattta gatgatacat tacttgatag ggataaagca     60

gtagaggcat tatttttaat tgttttagaa aagtgttatg aaaatgtaga tggtgcggct    120

aaaagcaaca tgttacagaa attcaaagaa tacgataaaa gagaatatgg cataagtaat    180

aaaacgacag ttttagaatc attgtttgat gaattcacgc caaggtatag attgccacgc    240

aattacatcc aagatttttg gaataataat ttccctagat gtttttcaat agaccaaaat    300

actattcatt tcttaaatca aataaagaag cactgtaaag ttggaattat aacaaatggc    360

tcaactcaga ggcaaaaagc taaaatattt aacacgaatt taaataagta ttttgaaaca    420

atcattattt ctgaagaagt gggatttagt aaacctgata aacgtatatt cgaactagca    480

ttaaataagt taaatttaca accggaaaat actttatttg ttggggatga cttagaaaag    540

gatattgctg gtcctcaaaa tgcaaatata aaaggtgtgt ggtttaaccc tcagaaaatc    600

aagaatacta ccaaaataca accatatgcc gagattaaca ctttggatag tttgttaagt    660

tatgttactc cacaatattt ttataacaag taa                                  693


<210>   3
<211>   236
<212>   PRT
<213>   Artificial Sequence

<220>
<223>   Bacillus saitens KCTC 13219BP


<400>   3
Met Lys Tyr Lys Val Ile Leu Phe Asp Val Asp Asp Thr Leu Leu Asp
1               5                   10                  15

Phe Pro Glu Thr Glu Arg His Ala Leu His Asn Ala Phe Val Gln Phe
            20                  25                  30
```

```
Asp Met Pro Thr Gly Tyr Asn Asp Tyr Leu Ala Ser Tyr Lys Glu Ile
        35                  40                  45

Ser Asn Gly Leu Trp Arg Asp Leu Glu Asn Lys Met Ile Thr Leu Ser
    50                  55                  60

Glu Leu Ala Val Asp Arg Phe Arg Gln Leu Phe Ala Leu His Asn Ile
65                  70                  75                  80

Asp Val Asp Ala Gln Gln Phe Ser Asp Val Tyr Leu Glu Asn Leu Gly
                85                  90                  95

Lys Glu Val His Leu Ile Glu Asp Ala Val Gln Leu Cys Glu Asn Leu
            100                 105                 110

Gln Asp Cys Lys Leu Gly Ile Ile Thr Asn Gly Tyr Thr Lys Val Gln
        115                 120                 125

Gln Ser Arg Ile Gly Asn Ser Pro Leu Cys Asn Phe Phe Asp His Ile
    130                 135                 140

Val Ile Ser Glu Glu Val Gly His Gln Lys Pro Ala Arg Glu Ile Phe
145                 150                 155                 160

Asp Tyr Ala Phe Glu Lys Phe Gly Ile Thr Asp Lys Ser Ser Val Leu
                165                 170                 175

Met Val Gly Asp Ser Leu Thr Ser Asp Met Lys Gly Gly Glu Asp Tyr
            180                 185                 190

Gly Ile Asp Thr Cys Trp Tyr Asn Pro Ser Leu Lys Glu Asn Gly Thr
        195                 200                 205

Asp Val Asn Pro Thr Tyr Glu Val Glu Ser Leu Leu Gln Ile Leu Glu
    210                 215                 220

Ile Val Glu Val Ala Glu Glu Lys Val Ala Ser Phe
225                 230                 235


<210>   4
<211>   711
<212>   DNA
<213>   Artificial Sequence

<220>
<223>   Bacillus saitens KCTC 13219BP


<400>   4
atgaaataca aagttatatt attcgacgta gatgatacat tattagattt ccctgaaacg      60

gaaagacacg cattacataa tgcgtttgta cagtttgata tgcctacagg gtataatgat     120

tatcttgcaa gctataaaga gattagtaat ggattatgga gagatttaga aaataaaatg     180

attacgctaa gtgaattagc agtagatcga tttagacaat tatttgcact tcataatata     240

gacgtagatg cacagcaatt tagtgatgta tatcttgaaa acttagggaa agaagtacat     300

cttatagaag acgcagtaca attatgtgaa aatctacaag attgcaagtt aggtattatt     360

acgaatggat atacgaaggt gcaacaatca agaatcggaa attcgccttt atgtaatttc     420
```

tttgatcaca ttgttatttc tgaagaagtt ggtcatcaaa aaccagcacg tgagattttt 480

gattatgcgt ttgagaagtt tgggattact gataaatcaa gcgtactaat ggttggagat 540

tcgttaactt ctgatatgaa aggcggagaa gattacggca ttgatacgtg ttggtataat 600

ccgagtttga aagaaaacgg gacagatgtt aacccgactt atgaagtgga gagtctgcta 660

caaattttag aaattgtaga agtggctgaa gaaaaggtag cttcatttta a 711

<210> 5
<211> 231
<212> PRT
<213> Artificial Sequence

<220>
<223> Bacillus saitens KCTC 13219BP

<400> 5
Met Lys Lys Tyr Lys Thr Leu Leu Phe Asp Val Asp Asp Thr Leu Leu
1 5 10 15

Asp Phe Gln Lys Ala Glu Lys Val Ala Leu Arg Val Leu Phe Glu Glu
20 25 30

Lys Gly Ile Pro Leu Thr Asp Glu Ile Glu Ala Arg Tyr Lys Lys Ile
35 40 45

Asn Lys Gly Leu Trp Asp Ala Phe Glu Lys Gly Glu Leu Ser Arg Asn
50 55 60

Glu Val Val Asn Thr Arg Phe Ser Leu Leu Phe Lys Glu Tyr Gly Glu
65 70 75 80

Glu Val Asn Gly Ile Leu Phe Glu Asn Asn Tyr Arg Asn Tyr Leu Glu
85 90 95

Glu Gly Asn Gln Leu Met Gln Gly Ala Phe Glu Phe Ile Asn Gln Ile
100 105 110

Gln Gly Glu Tyr Glu Leu Tyr Ile Val Thr Asn Gly Val Ser Lys Thr
115 120 125

Gln Asp Lys Arg Leu Arg Asn Ala Gly Leu His Ser Leu Phe Lys Asp
130 135 140

Val Phe Val Ser Glu Asp Thr Gly Phe Gln Lys Pro Met Lys Glu Tyr
145 150 155 160

Phe Asp Tyr Val Phe Glu Arg Ile Pro Asn Phe Ala Pro Glu Glu Gly
165 170 175

Leu Ile Ile Gly Asp Ser Leu Ser Ala Asp Ile Lys Gly Gly Tyr Val
180 185 190

Ala Gly Ile Asp Thr Cys Trp Phe Asn Pro Glu Arg Lys Leu Asn Asp
195 200 205

Ser Gly Ile Ile Pro Thr Tyr Glu Val His Asn Phe Glu Glu Leu Glu
210 215 220

Ala Leu Leu Lys Gln His Val
225 230

<210> 6
<211> 696
<212> DNA
<213> Artificial Sequence

<220>
<223> Bacillus saitens KCTC 13219BP

<400> 6
atgaaaaaat ataaaacatt gttatttgat gtagatgata cattactaga cttccaaaag 60

gctgaaaaag tggctttacg ggtacttttt gaagagaagg gaatcccttt aacagacgag 120

atagaggctc gttataaaaa aataaataaa ggtctttggg atgcttttga aaaaggtgaa 180

ctatcacgca atgaagttgt aaatacacga ttctctctgt tgtttaaaga gtatggagaa 240

gaagtaaatg gaatattatt cgaaaataat tatcgtaact acttagaaga aggaaatcaa 300

ctcatgcaag gtgcatttga atttataaat caaatccaag gggagtatga attgtatata 360

gtgacaaatg gtgtttctaa gacgcaagat aaacgtttgc gcaatgcagg gttacattca 420

ttatttaaag atgtctttgt ttcggaagat acaggttttc aaaaaccgat gaaagagtat 480

tttgattatg tttttgaacg gattcctaac tttgcacctg aagaagggct gattattggt 540

gattcattaa gcgctgatat taaaggtgga tatgtagcgg gaattgatac ttgttggttt 600

aatccagaaa ggaaattaaa tgatagtggg attataccga catatgaagt gcataatttt 660

gaggagttag aagcattatt gaagcagcat gtgtaa 696

<210> 7
<211> 38
<212> DNA
<213> Artificial Sequence

<220>
<223> primer

<400> 7
aagaaggaga tataccatga tgggttataa agcgatgc 38

<210> 8
<211> 41
<212> DNA
<213> Artificial Sequence

<220>
<223> primer

<400> 8

gcattatgcg gccgcaagct ttacttgtta taaaaatatt g 41

<210> 9
<211> 40
<212> DNA
<213> Artificial Sequence

<220>
<223> primer

<400> 9
aagaaggaga tataccatga aatacaaagt tatattattc 40

<210> 10
<211> 41
<212> DNA
<213> Artificial Sequence

<220>
<223> primer

<400> 10
gcattatgcg gccgcaagct ttaaaatgaa gctacctttt c 41

<210> 11
<211> 37
<212> DNA
<213> Artificial Sequence

<220>
<223> primer

<400> 11
aagaaggaga tataccatga aaaaatataa aacattg 37

<210> 12
<211> 40
<212> DNA
<213> Artificial Sequence

<220>
<223> primer

<400> 12
gcattatgcg gccgcaagct ttacacatgc tgcttcaata 40

**Description**

BACKGROUND

1. Field

The present disclosure relates to *Bacillus saitens* (KCTC 13219BP) having activity in reducing a concentration of a fluorine-containing compound in a sample, a recombinant microorganism including a gene from *B. saitens* (KCTC 13219BP), a composition including the recombinant microorganism for use in reducing a concentration of a fluorine-containing compound in a sample, and a method of reducing a concentration of a fluorine-containing compound in a sample.

2. Description of the Related Art

The emission of greenhouse gases, which have accelerated global warming, is a serious environmental problems, and regulations to reduce and prevent the emissions of greenhouse gases have been tightened. Among the greenhouse gases, fluorinated gases (F-gases), such as perfluorocarbons (PFCs), hydrofluorocarbon (HFCs), or sulfur hexafluoride ($SF_6$) show low absolute emission but have a long half-life and a very high global warming potential, resulting in significantly adverse environmental impacts. The amount of F-gases emitted from the semiconductor and electronics industries, which are major causes of F-gas emission, has exceeded the assigned amount of greenhouse gas emissions and continues to increase. Therefore, costs required for decomposition of greenhouse gases and greenhouse gas emission allowances are increasing every year.

A pyrolysis or catalytic thermal oxidation process has generally been used in the decomposition of F-gases. However, this process has disadvantages of limited decomposition rate, emission of secondary pollutants, and high cost. However, biological decomposition of F-gases would allow F-gases to be treated in a more economical and environmentally-friendly manner.

Therefore, there is a need to develop new microorganisms and methods for the biological decomposition of F-gases. This invention provides such microorganisms and methods.

SUMMARY

Provided herein is a microorganism referred to as *Bacillus saitens* (KCTC 13219BP) having activity in reducing a concentration of a fluorine-containing compound in a sample.

Also provided is a microorganism having a genetic modification that increases the level of a polypeptide having a sequence identity of about 90% or more with respect to an amino acid sequence of SEQ ID NO: 1, 3, or 5, as well as a method of preparing such microorganism.

Provided is a composition for use in reducing a concentration of a fluorine-containing compound in a sample, the composition including *B. saitens* (KCTC 13219BP) or the recombinant microorganism having a genetic modification that increases the level of a polypeptide having a sequence identity of about 90% or more with respect to an amino acid sequence of SEQ ID NO: 1, 3, or 5.

Provided is a method of reducing a concentration of a fluorine-containing compound in a sample, the method including contacting a sample including a fluorine-containing compound with *B. saitens* (*KCTC 13219BP*) or the recombinant microorganism having a genetic modification that increases a level of a polypeptide having a sequence identity of about 90% or more with respect to an amino acid sequence of SEQ ID NO: 1, 3, or 5, so as to reduce the concentration of the fluorine-containing compound in the sample.

Provided is a vector comprising a promoter operably linked to a nucleic acid sequence comprising SEQ ID NO: 2, 4, or 6.

BRIEF DESCRIPTION OF THE DRAWINGS

These and/or other aspects will become apparent and more readily appreciated from the following description of the embodiments, taken in conjunction with the accompanying drawings in which:

FIG. 1 is a vector map of a pET-BS DEH vector;
FIG. 2 is a schematic diagram of a reactor used in Example 3;
FIG. 3 is a graph showing decomposition rates of $CF_4$ when a strain of *B. saitens* is brought into contact with a fluorine-containing compound;
FIG. 4 is a graph showing decomposition rates of $CF_4$ when a strain of BL21/pET-BS01766 is brought into contact with a fluorine-containing compound;

FIG. 5 is a graph showing decomposition rates of $CF_4$ when a strain of *Bacillus cereus* is brought into contact with a fluorine-containing compound; and

FIG. 6 is a schematic diagram for decomposing $CF_4$ by applying a gas-phase circulation process using a microorganism.

DETAILED DESCRIPTION

Reference will now be made in detail to embodiments, examples of which are illustrated in the accompanying drawings, wherein like reference numerals refer to like elements throughout. In this regard, the present embodiments are exemplary and may have different forms. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

The term "increase in the level of a polypeptide" as used herein may refer to a detectable increase in the amount or concentration of a polypeptide in a cell. The term "increase in the level of a polypeptide " may refer to a level of a polypeptide in a cell, such as a genetically modified cell or a recombinant microorganism, that is higher than the level of the polypeptide in a comparative cell of the same type, such as a cell that does not have a given genetic modification. Any increase of any amount is encompassed. The increase in the level of a polypeptide of a given cell (e.g., a cell with a given genetic modification) may be, for instance, about 5% or greater, about 10% or greater, about 15% or greater, about 20% or greater, about 30% or greater, about 50% or greater, about 60% or greater, about 70% or greater, or about 100% or greater, than a comparative cell (e.g., a cell of the same type without the genetic modification).

The increase in level of polypeptide may be achieved by an increase in expression of a gene encoding the polypeptide. The increase in the expression may be achieved by introduction of a polynucleotide encoding the polypeptide to a cell; an increase in the copy number of the gene encoding the polypeptide, or a modification of a regulatory region of the polynucleotide encoding the polypeptide that increases expression of the polynucleotide. The polynucleotide encoding the polypeptide may be operably linked to a regulatory sequence that allows expression thereof, for example, a promoter, an enhancer, a polyadenylation region, or a combination thereof. The polynucleotide which is introduced into the cell or whose copy number is increased in the cell may be endogenous or heterologous to the cell. The term "endogenous gene" refers to a gene which is included in a microorganism prior to introducing the genetic modification (e.g., a native gene). The term "heterologous" refers to a gene that is "foreign," or "not native" to the species. In either case, a polynucleotide or gene that is introduced into a cell is referred to as "exogenous" and an exogenous gene or polynucleotide may be endogenous or heterologous with respect to a cell into which the gene is introduced. Thus, the microorganism into which the polynucleotide encoding the polypeptide is introduced may be a microorganism that already includes the gene encoded by the polynucleotide (e.g., the gene or polynucleotide is endogenous to the microorganism). Alternatively, the microorganism can be without a copy of the gene prior to its introduction (e.g., the polynucleotide or gene is heterologous to the microorganism)

The term "increase of copy number" as used herein may be caused by introduction of an exogenous polynucleotide or amplification of an endogenous gene. In an embodiment, the increase of copy number may be caused by a genetic modification such as introduction of a gene that does not exist in a non-engineered microorganism. In other words, the microorganism, such as a recombinant microorganism, can comprise more copies of the gene, or can comprise an exogenous gene (e.g., a heterologous gene). The introduction of such a gene may be mediated by a vehicle such as a vector. The introduction may be achieved by transient introduction in which the gene is not integrated into a genome, or by insertion of the gene into the genome. The introduction may be achieved by, for example, introducing a vector into the cell, and then replicating the vector in the cell, wherein the vector includes a polynucleotide encoding a target polypeptide, or by integrating the polynucleotide into the genome.

The introduction of the gene may be performed by any known method in the art, such as transformation, transfection, or electroporation. The gene may be introduced via a vehicle or may be introduced by itself . The term "vehicle" as used herein may refer to a nucleic acid molecule that is able to deliver other nucleic acids linked thereto. As a nucleic acid sequence mediating introduction of a specific gene, the vehicle as used herein may be construed to be interchangeable with a vector, a nucleic acid structure, and a cassette. The vector may include, for example, a plasmid vector or a virus-derived vector. The plasmid may include a circular double-stranded DNA ring linkable with another DNA. The vector may include, for example, a plasmid expression vector, a virus expression vector, such as a replication-defective retrovirus, adenovirus, and adeno-associated virus, or a combination thereof.

The term "parent cell" as used herein refers to an original cell, for example, a non-genetically modified cell of the same type as the genetically engineered microorganism. In regard to a particular genetic modification, the "parent cell" may be a cell that lacks the particular genetic modification, but is identical in all other respects. Thus, the parent cell may be a cell that is used as a starting material to produce a genetically engineered microorganism having increased activity of a given protein (for example, a protein having a sequence identity of about 90% or more to a dehalogenase). The same comparison may be also applied to other genetic modifications.

The term "gene" as used herein may refer to a polynucleotide expressing a specific protein. A gene may include regulatory sequences such as a 5' non-coding sequence and/or a 3' non-coding sequence, or may be free from regulatory sequences.

The term "sequence identity" of a nucleic acid or polypeptide as used herein refers to a degree of identity between nucleotides or amino acid residues of sequences obtained after the sequences are aligned so as to best match in certain comparable regions. The sequence identity is a value measured by comparing two sequences in certain comparable regions via optimal alignment of the two sequences, in which portions of the sequences in the certain comparable regions may be added or deleted compared to reference sequences. A percentage of sequence identity may be calculated by, for example, comparing two optimally aligned sequences in the entire comparable regions, determining the number of locations in which the same amino acids or nucleic acids appear to obtain the number of matching locations, dividing the number of matching locations by the total number of locations in the comparable regions (that is, the size of a range), and multiplying a result of the division by 100 to obtain the percentage of the sequence identity. The percentage of the sequence identity may be determined using a known sequence comparison program, for example, BLASTN(NCBI), BLASTP(NCBI), CLC Main Workbench (CLC bio), or MegAlign™ (DNASTAR Inc).

The term "genetic modification" as used herein may refer to an artificial modification in a constitution or structure of a genetic material of a cell. The "genetic modification" include a genetic modification caused by introducing into the microorganism at least one recombinant nucleic acid encoding a nuclease targeting specific polynucleotide. Non-limiting examples of a nuclease useful for generating mutants in an endogenous gene of the invention includes a meganuclease, a zinc finger nuclease (ZFN), a transcription activator-like effector nuclease (TALEN), and/or a clustered regularly interspaced, short palindromic repeat (CRISPR) associated (Cas) nuclease. Clustered regularly interspaced, short palindromic repeat (CRISPR) associated (Cas) nucleases are useful for modifying endogenous polynucleotides. newly engineered CRISPR-Cas nucleases are not required for each use. The requirements for cleavage and DNA modification using CRISPR-Cas systems are well known and routine. Type II CRISPR systems are the monst commonly used for genome editing. The bacterial Type II CRISPR systems comprise two RNA components, a CRISPR RNA (crRNA) and a transactivating RNA (tracrRNA). To facilitate use in genome engineering applications, these two RNA components may be combined into a single RNA that is referred to as a guide RNA (gRNA or sgRNA). The most commonly used genome engineering system is the CRISPR-Cas9 system derived from *Streptococcus pyogenes.*

The symbol "%" as used herein indicates w/w%, unless otherwise stated.

An aspect of the present invention provides a polypeptide having a sequence identity of about 90% or more with respect to an amino acid sequence of SEQ ID NO: 1, 3, or 5.

The polypeptide may include a detectable label attached thereto. The detectable label may be a fluorescent material, a material having a specific binding ability, or a material capable of binding to the material having a specific binding ability.

The polypeptide may be dehalogenase. The term "dehalogenase" as used herein may refer to an enzyme that catalyzes the removal of a halogen atom from a substrate. The dehalogenase may be 4-chlorobenzoate dehalogenase, 4-chlorobenzoyl-CoA dehalogenase, dichloromethane dehalogenase, fluoroacetate dehalogenase, haloacetate dehalogenase, (R)-2-haloacid dehalogenase, (S)-2-haloacid dehalogenase, haloalkane dehalogenase, halohydrin dehalogenase, or tetrachloroethene reductive dehalogenase. For example, the dehalogenase may belong to a haloacid dehalogenase superfamily. The haloacid dehalogenase superfamily may be EC 3.8.1.2. The polypeptide may have a sequence identity of about 90% or more, 95% or more, 96% or more, 97% or more, 98% or more, or 99% or more, with respect to an amino acid sequence of SEQ ID NOs: 1, 3, or 5. The polypeptide may include an amino acid sequence selected from SEQ ID NOs: 1, 3, or 5.

Another aspect of the invention provides a polynucleotide including a nucleotide sequence encoding a polypeptide having a sequence identity of about 90% or more with respect to an amino acid sequence of SEQ ID NO: 1, 3, or 5. In an embodiment, the polynucleotide sequence comprises SEQ ID NO: 2, 4, or 6.

The polynucleotide may be in a vector. The vector may be an expression vector, which is configured to express a foreign gene inserted into the vector in a host organism. The vector may include an origin, a promoter, a cloning site, a marker, or a combination thereof. The vector may be, for example, a plasmid. The polynucleotide may be inserted into a cloning site in association with an open reading frame, so as to be expressed in a host organism. In one embodiment, the vector includes a promoter operably linked to a nucleic acid sequence comprising SEQ ID NO: 2, 4, or 6.

Another aspect of the invention provides a microorganism including a genetic modification that increases a level of a polypeptide having a sequence identity of about 90% or more with respect to an amino acid sequence of SEQ ID NO: 1, 3, or 5.

The genetic modification may include an increase in the copy number of a gene encoding the polypeptide. The genetic modification may include introduction of an exogenous polynucleotide encoding the polypeptide, such as by transformation, transfection, or electroporation of the polynucleotide encoding the polypeptide. The microorganism may be a microorganism to which the gene encoding the polypeptide is introduced. The gene may have a sequence identity of 90% or more, 95% or more, 96% or more, 97% or more, 98% or more, or 99% or more, with respect to a nucleotide sequence of SEQ ID NO: 2, 4, or 6. The microorganism may belong to the genus *Escherichia, Bacillus, Pseudomonas, Xanthobacter,* or *Saccharomyces.* In an embodiment, the microorganism may be E. coli or *B. saitens.*

Another aspect of the invention provides a method for preparing the inventive microorganisms described herein, the method comprising introducing into a microorganism a genetic modification that increases the level of a polypeptide comprising the amino acid sequence of SEQ ID NO: 1, 3, or 5. In an embodiment the method comprises introducing into the microorganism an exogenous, optionally heterologous, nucleic acid that encodes the polypeptide. In an embodiment the exogenous, optionally heterologous, nucleic acid comprises SEQ ID NO: 2, 4, or 6 or has a sequence identity of 90% or more, 95% or more, 96% or more, 97% or more, 98% or more, or 99% or more thereto. In certain embodiments the microorganism for the method for preparing the inventive microorganism is selected from the genus *Xanthobacter, Agrobacterium, Corynebacterium, Rhodococcus, Mycobacterium, Klebsiella,* or *Escherichia.*

The microorganism may have activity in reducing a concentration of the "fluorine-containing compound" in a sample. The fluorine-containing compound may be $CH_3F$, $CH_2F_2$, $CHF_3$, $CF_4$, or a mixture thereof.

Another aspect of the invention provides a composition for use in reducing a concentration of a fluorine-containing compound in a sample, the composition including *Bacillus saitens* (KCTC 13219BP) or any microorganism described herein. In certain embodiments the composition may comprise a fluorine-containing compound, such as those described herein.

The microorganism is the same as described above. Without wishing to be bound by any particular mechanism of action, it is believed the reduced concentration may be achieved in a way that the polypeptide cleaves a C-F bond of the fluorine-containing compound, the fluorine-containing compound is converted into a different substance, or the fluorine-containing compound is accumulated in a cell.

The sample may be a liquid sample, a gaseous sample, or a combination thereof. The sample may be free of the microorganism. The sample may be industrial sewage or waste gas. For example, the sample may be sludge.

Another aspect of the invention provides a method of reducing a concentration of a fluorine-containing compound in a sample, the method including contacting a sample including a fluorine-containing compound with *Bacillus saitens* (KCTC 13219BP) or a microorganism described herein (e.g., comprising a genetic modification that increases the level of a polypeptide having a sequence identity of about 90% or more with respect to an amino acid sequence of SEQ ID NO: 1, 3, or 5), so as to reduce the concentration of the fluorine-containing compound in the sample.

Bacteria of the genus *Bacillus* are aerobic or facultatively anaerobic bacteria, and are generally Gram-positive spore-forming bacteria. The *Bacillus saitens* may be a strain harvested from sewage sludge. In one embodiment, the strain of *B. saitens* is KCTC 13219BP.

The fluorine-containing compound referred to herein may be an alkane compound having 1 to 12 carbon atoms substituted with at least one fluorine. The term "fluorine-containing compound" as used herein may be represented by Formula 1 or Formula 2:

<Formula 1> $C(R_1)(R_2)(R_3)(R_4)$

<Formula 2> $(R_5)(R_6)(R_7)C-[C(R_{11})(R_{12})]n-C(R_8)(R_9)(R_{10})$.

In Formula 1 $R_1$, $R_2$, $R_3$, and $R_4$ may each independently be fluorine (F), chlorine (Cl), bromine (Br), iodine (I), or hydrogen (H), wherein at least one selected from $R_1$, $R_2$, $R_3$, and $R_4$ is F. In certain embodiments, the fluorine-containing compound may be $CH_3F$, $CH_2F_2$, $CHF_3$, $CF_4$, or a mixture thereof.

In Formula 2, n may be an integer from 0 to 10, and when n is equal or larger than 2, each of $R_{11}$ is identical to or different from each other and each of $R_{12}$ is identical to or different from each other, $R_5$, $R_6$, $R_7$, $R_8$, $R_9$, $R_{10}$, $R_{11}$, and $R_{12}$ may each independently be F, Cl, Br, I, or H, wherein at least one selected from $R_5$, $R_6$, $R_7$, $R_8$, $R_9$, $R_{10}$, $R_{11}$, and $R_{12}$ is F.

In an embodiment, of Formula 2, n may be an integer from 0 to 3, an integer from 0 to 4, an integer from 0 to 5, or an integer from 0 to 7.

The sample may be a liquid sample, a gaseous sample, or a combination thereof. The sample may be industrial waste water or waste gas. For example, the sample may be sludge. The sample may be free of *B. saitens* or the microorganism prior to contacting the sample with such microorganism.

Contacting the sample with the microorganism may be performed in a liquid phase, or a gaseous phase. The contacting may include culturing the *B. saitens* (e.g., KCTC 13219BP), the microorganism, or a combination thereof, in the presence of the fluorine-containing compound or sample comprising same. The contacting may be performed in an air-tight sealed container. The contacting may be performed when the growth stage of the *B. saitens* (e.g., KCTC 13219BP) or the microorganism is in an exponential phase or a stationary phase. The culturing may be performed under aerobic or anaerobic conditions. Alternatively, the contacting may be performed under conditions where the *B. saitens* (e.g., KCTC 13219BP), the microorganism, or a combination thereof may survive in the closed container. Such conditions appropriate for survival of the *B. saitens* (e.g., KCTC 13219BP), the microorganism, or a combination thereof may include conditions where the *B. saitens* (e.g., KCTC 13219BP), the microorganism, or a combination thereof may proliferate or may be allowed to be in a resting state.

The contacting may include passive contacting and/or active contacting. The active and passive contacting refers to contacting with or without external driving force, respectively. The contacting may be achieved in a way that the fluorine-containing compound is injected in the form of bubbles into a solution containing the *B. saitens* (e.g., KCTC 13219BP) and/or the microorganism, or is sprayed. For example, the contacting may be achieved by blowing the sample into a medium or a culture broth. By way of further illustration, for the injection of the sample, the sample may be blown from the bottom of the medium or the culture broth to the top thereof. The injection of the sample may be achieved by making droplets of the sample. The contacting may be performed in a batch or continuous manner. The contacting may be performed repeatedly, such as two or more times, for example, three times, five times, or ten times or more. The contacting may be continued or repeated until the fluorine-containing compound is reduced to a desired concentration.

In some embodiments, the *B. saitens* (e.g., KCTC 13219BP), the microorganism, or a combination thereof may be in the form of a thin film layer, such as a liquid thin film layer. The fluorine-containing compound or sample comprising same may be in the form of a gaseous thin film layer. The liquid thin film layer formed by the *B. saitens* (e.g., KCTC 13219BP), the microorganism, or a combination thereof and the gaseous thin film layer formed by the fluorine-containing compound may contact each other according to the method.

In an embodiment, the method can comprise subjecting the *B. saitens* (e.g., KCTC 13219BP), the microorganism, or a combination thereof to a circulation process, so that the contact area or the time of contact of the microorganism with the fluorine-containing compound or sample comprising same may increase. The circulation process may increase the mass transfer coefficient (KLa) value, as well as increase the amount and/or rate of decomposition of the fluorine-containing compound.

The contacting of the inventive method may further include, using an exhaust gas decomposition device including one or more reactors each of which includes at least a first inlet and a first outlet. Such a method can involve injecting the sample into the exhaust gas decomposition device and injecting *B. saitens* (KCTC 13219BP) or the microorganism into the device through the at least one first inlet (the microorganism and sample can, for instance, be introduced through the same inlet or different inlets), so that *B. saitens* (KCTC 13219BP) or the microorganism may contact the sample and the resulting mixture may be discharged through the first outlet.

In some embodiments, the exhaust gas decomposition device may include a second inlet and a second outlet, and the sample may be injected through the second inlet and discharged through the second outlet. In such a configuration, the *B. saitens* (KCTC 13219BP) or the microorganism can move in a direction opposite to a direction in which the sample moves, for instance, by supplying the microorganism through a different inlet and discharging from a differnet outlet than the sample. In still other embodiments, a fluid thin film including *B. saitens* (KCTC 13219BP) or the microorganism may be formed on an inner wall of the one or more reactors.

The exhaust gas decomposition device used in the method may further include a first circulation line for re-supplying at least a portion of a fluid to the at least one first inlet, wherein the fluid contains *B. saitens* (KCTC 13219BP) or the microorganism discharged through the first outlet. The sample including the fluorine-containing compound may remain inside the one or more reactors, or may be circulated. In addition, the one or more reactors of the exhaust gas decomposition device may further include a second inlet and a second outlet, wherein the sample may be supplied into the one or more reactors through the second inlet and discharged to the outside of the one or more reactors through the second outlet. The sample may, then, move along a second direction within the one or more reactors, wherein the second direction may be different from, (e.g., opposite) the direction in which *B. saitens* (KCTC 13219BP) or the microorganism moves. In addition, in at least one of a fluid collection zone at the bottom of the inside of the one or more reactors and a fluid reaction zone at the top of the inside of the one or more reactors of the exhaust gas decomposition device, the fluid including *B. saitens* (KCTC 13219BP) or the recombinant and the sample including the fluorine-containing compound may contact each other, thereby decomposing the fluorine-containing compound. In the fluid reaction zone, a fluid thin film including the fluid containing *B. saitens* (KCTC 13219BP) or the microorganism may contact a fluid including the sample.

The exhaust gas decomposition device used in the method may further include a structure inside the one or more reactors, wherein the structure may be configured to increase the contact area between the fluid including *B. saitens* (KCTC 13219BP) or the microorganism and the sample including the fluorine-containing compound. Any structure configured to a contact area between the fluid including *B. saitens* (KCTC 13219BP) or the recombinant microorganism and the sample including the fluorine-containing compound may be included. For example, the structure comprise a packing material or a reflux tube, but is not limited thereto. The 'packing material' may be inert solid material. The packing material may have a various shape. The packing material may be the same material used in the packing of a packed bed tower. The packing material may be made of plastic, magnetic material, steel or aluminium. The packing material may have very thin thickness. The packing material may have a ring shape such as rashing ring, pall ring, and berl saddle, a saddle type, and protrusion type. The packing material may be irregularly packed in the packed bed reactor. The packing material may efficiently increase contact between the fluorine-containing compound with a microorganism present in a liquid. The time or opportunity to contact between the fluorine-containing compound with a microorganim can be maximized by forming a thin film of a microorganims on the surface of the packing material as well as on the inner surface of the reactor. In addition, the at least one first inlet may be connected to the fluid reaction zone at the top of the inside of the one or more reactors in the exhaust gas decomposition device, to thereby supply the fluid including *B. saitens* (KCTC 13219BP) or the microorganism through the at least one first inlet.

According to an aspect of the method, the fluid including *B. saitens* (KCTC 13219BP) or the microorganism may be collected in the fluid collection zone at the bottom of the inside of the one or more reactors in the exhaust gas decomposition device. The sample including the fluorine-containing compound supplied into the one or more reactors through the second inlet may pass through, in the form of bubbles, the collected fluid including *B. saitens* (KCTC 13219BP) or the recombinant to be transferred to the fluid reaction zone at the top of the inside of the one or more reactors, and then, may be discharged to the outside of the one or more reactors through the second outlet.

In the exhaust gas decomposition device, the aspect ratio of the height H of the one or more reactors to the diameter D of the one or more reactors (H/D) may be 2 or more, 5 or more, 10 or more, 15 or more, 20 or more, or 50 or more.

Furthermore, the exhaust gas decomposition device may be arranged in a way that the side-wall of one or more reactors, or some other internal surface thereof, is tilted or inclined at an angle of less than or greater than 90° relative to the surface of the earth, i.e. the horizontal plane, and/or a plane defined by the vector of the gravitational force of the earth. For example, the side-wall or other internal surface thereof can be tilted or inclined in a range of about 30° to less than 90° (or greater than 90° to about 150°), about 70° to less than 90° (or greater than 90° to about 110°), about 80° to less than 90° (or greater than 90° to about 100°), or about 50° to less than 90°, with respect to the surface of the earth, i.e. the horizontal plane, and/or a plane defined by the vector of the gravitational force of the earth.

Regarding the method, the one or more reactors in the exhaust gas decomposition device may rotate. The fluid containing *B. saitens* (KCTC 13219BP) or the microorganism may be liquid, and the sample including the fluorine-containing compound may be gas.

Hereinafter, the present invention will be described in more detail with reference to Examples. However, these Examples are provided for illustrative purposes only.

### Example 1: Selection of strain of *Bacillus saitens* and decomposition of fluorine-containing compound using the strain

In Example 1, a microorganism capable of reducing a concentration of $CF_4$ in waste water of a semiconductor factory was selected.

Sludge in waste water discharged from Samsung Electronics Plant (Giheung, Korea) was smeared on an agar plate including a carbon-free medium (supplemented with 0.7 g/L of $K_2HPO_4$, 0.7 g/L of $MgSO_4 \cdot 7H_2O$, 0.5 g/L of $(NH_4)2SO_4$, 0.5 g/L of $NaNO_3$, 0.005 g/L of NaCl, 0.002 g/L of $FeSO_4 \cdot 7H_2O$, 0.002 g/L of $ZnSO_4 \cdot 7H_2O$, 0.001 g/L of $MnSO_4$, and 15 g/L of Agar), and the agar plate was put in a GasPak™ Jar (BD Medical Technology). The jar was filled with 99.9 v/v% of $CF_4$, and then, was sealed for standing culture at a temperature of 30°C under anaerobic conditions. Single colonies formed on the agar plate after the culture were cultured again using a high throughput screening (HTS) system (Thermo Scientific/Liconic/Perkin Elmer). Each of the cultured single colonies was then inoculated on a 96-well microplate, each well of which contained 100 μL of an LB medium. The 96-well microplate was subjected to standing culture at a temperature of about 30°C for 96 hours under aerobic conditions. Meanwhile, the growth ability of the single colonies was observed by measuring the absorbance thereof at 600 nm every 12 hours. The LB medium used herein included 10 g/L of tryptone, 5 g/L of yeast extract, and 10 g/L of NaCl.

The top 2% of strains showing excellent growth ability were selected, and then inoculated in a glass serum bottle (volume of 75 mL) containing 10 mL of an LB medium to have $OD_{600}$ of 0.5. The glass serum bottle was sealed, and $CF_4$ was injected thereto using a syringe to have 1,000 ppm of $CF_4$ gas. The glass serum bottle was incubated in a shaking incubator for 4 days at a temperature of 30°C while being stirred at a speed of 230 rpm. Then, an amount of $CF_4$ in a head space of the glass serum bottle was analyzed.

For the analysis, 0.5 ml of the headspace gas in the glass serum bottle was collected using a syringe and injected into gas chromatography (GC, Agilent 7890, Palo Alto, CA, USA). The injected headspace sample was separated through a CP-PoraBOND Q column (25 m length, 0.32 mm i.d., 5 um film thickness, Agilent), and changes in the $CF_4$ concentration were analyzed by a Mass Selective Detector (MSD) (Agilent 5973, Palo Alto, CA, USA). Helium was used as carrier gas, and applied to the column at a flow rate of 1.5 ml/min in the gas chromatography column. GC conditions were as follows: an inlet temperature was 250°C and an initial temperature was maintained at 40°C for 2 minutes and raised to 290°C at a rate of 20°C/min. Mass spectrometry (MS) conditions were as follows: an ionization energy was 70 eV, an interface temperature was 280°C, an ion source temperature was 230°C, and a quadrupole temperature was 150°C. As a control group, the headspace sample having the $CF_4$ concentration of 1,000 ppm was incubated in the same manner in a glass serum bottle containing no cells, followed by being subjected to the measurement.

Consequently, compared to the control group having no cells, the concentration of $CF_4$ was reduced by 12.48% in a separated microorganism among the tested strains. The microorganism exhibited decomposition activity of 0.03144 g/kg-cell/. To identify the selected strains, genome sequences thereof were analyzed.

A genome obtained by assembling 6 contigs by next generation sequencing had a final size of 5.2 Mb, and as a result of gene annotation, a total of 5,210 genes were found to be present. As a result of the phylogenetic tree analysis performed on each contig, it was confirmed that the microorganism belonged to genus *Bacillus.* The genome sequence of the selected microorganism has about 93 % amino acid sequence identity with that of Bacillus thuringiensis. Further, about 98% of the genome sequence of the microorganim was given an annotation after annotation analysis.

The separated microorganism was newly named as *Bacillus saitens,* deposited at the Korean Collection for Type Culture (KCTC), which is an international depository authority under the Budapest Treaty, on February 24, 2017, and assigned the number of KCTC 13219BP.

**Example 2: Preparation of microorganism including gene derived from strain of *B. saitens,* and decomposition of fluorine-containing compound using the microorganism**

**1. Preparation of microorganism**

By the genomic sequence analysis of the strain of *B. saitens* identified as described in Example 1, genes presumed to encode dehalogenase, such as GENE_01070 (SEQ ID NO: 2), GENE_01766 (SEQ ID NO: 4), and GENE_03901 (SEQ ID NO: 6), were selected.

B. *saitens* was cultured overnight in an LB medium while being stirred at a temperature of 30°C at a speed of 230 rpm, and genomic DNA thereof was isolated using a total DNA extraction kit (Invitrogen Biotechnology). PCR was performed using the genomic DNA as a template and a set of primers having nucleotide sequences shown in Table 1, so as to amplify and obtain GENE_01070, GENE_01766, and GENE_03901. The genes thus amplified were each independently ligated with a pETDuet-1 vector (Novagen, Cat. No. 71146-3), using restriction enzymes, such as Ncol and HindIII, by using an InFusion Cloning Kit (Clontech Laboratories, Inc.), so as to prepare three types of pET-BS DEH vectors. FIG.1 is a vector map of the pET-BS DEH vectors. Here, GENE_01070, GENE_01766, and GENE_03901 had nucleotide sequences of SEQ ID NOs: 2, 4, and 6, respectively, and encoded amino acid sequences of SEQ ID NOs: 1, 3, and 5, respectively.

Next, each of the three prepared pET-BS DEH vectors (pET-BS01070 vector, pET-BS01766 vector, and pET-BS03901 vector) were introduced to E. coli BL21 by a heat shock method, and then, cultured in an LB plate agar containing 100 μg/mL of ampicillin. Strains showing ampicillin resistance were selected. Finally, three strains thus selected were designated as recombinant E. *coli* BL21/pET-BS01070, E. *coli* BL21/pET-BS01766, and E. *coli* BL21/pET-BS03901, respectively.

[Table 1]

| BS gene | Primer sequence (SEQ ID NO) |
|---|---|
| BS01070 | Forward: SEQ ID NO: 7<br>Reverse: SEQ ID NO: 8 |
| BS01766 | Forward: SEQ ID NO: 9<br>Reverse: SEQ ID NO: 10 |
| BS03901 | Forward: SEQ ID NO: 11<br>Reverse: SEQ ID NO: 12 |

**2. Decomposition of fluorine-containing compound using *E. coli* including gene introduced thereto**

In this section, was examined whether the three kinds of recombinant *E. coli* BL21/pET-SF3 DEH strains prepared in section (1) affect removal of CF4 in a sample.

In detail, the strains of *E. coli* BL21/pET-BS01070, *E. coli* BL21/pET-BS01766, and *E. coli* BL21/pET-BS03901 were cultured in an LB medium while being stirred at a temperature of 30°C at a speed of 230 rpm. At an $OD_{600}$ of about 0.5, 0.2 mM of IPTG was added thereto, followed by culturing at a temperature of 20°C under stirring at a speed of 230 rpm overnight. Each of the cells was harvested and suspended in a new LB medium to a cell density of $OD_{600}$ of 3.0. 10 ml of each cell suspension was added to a 60 ml-serum bottle, and then, the serum bottle was sealed. The LB medium used herein has the same composition as in Example 1.

Next, gas-phase $CF_4$ was injected through a rubber stopper of a cap of the serum bottle using a syringe to its headspace concentration of 1,000 ppm. Then, the serum bottle was incubated for three days while being stirred at a temperature of 30°C at a speed of 230 rpm. Here, the experiment was performed in triplicate. Following the culture, a headspace concentration of CF4 in the serum bottle was analyzed under the same conditions as in Example 1.

Table 2 shows percentages of residual $CF_4$ in the samples when the recombinant *E. coli* BL21/pET-BS DEH strains were cultured under the conditions as above. As shown in Table 2, the recombinant *E.* coli strains introduced with GENE_01070, GENE_01766, and GENE_03901 showed about 5.1% decrease, about 7.9% decrease, and about 7.1% decrease in the headspace concentrations of $CF_4$, compared to a control group introduced with an empty vector.

[Table 2]

| Strain of recombinant microorganism | Residual $CF_4$ (%) |
|---|---|
| Control (empty vector) | 100.00 |
| BS01070 | 94.90 |
| BS01766 | 92.11 |
| BS03901 | 92.87 |

**Example 3: Decomposition of fluorine-containing compound by a circulation process**

As shown in FIG. 2, 40 ml of an LB medium and gas-phase $CF_4$ at a concentration of 1,000 ppm were added to a glass Dimroth coil reflux condenser (a reactor length: 350 mm, an exterior diameter: 35 mm, and an interior volume: 200 mL) that was sterilized and vertically oriented, and the LB medium was circulated. The LB medium was first supplied to an inlet at an upper portion of the condenser, flowed through an inner wall of the condenser, and then, discharged to an outlet at a lower portion of the condenser. The discharged LB medium was re-supplied to the inlet along a circulation line. Although not shown in FIG. 2, to maintain a temperature of the condenser, a screwed pipe inside the condenser was connected to a constant temperature zone having a temperature of 30°C. Here, the LB medium was maintained at a circulation rate of 4 mL/min. After 48 hours, the amount of the gas-phase $CF_4$ in the condenser was confirmed by GC-MS. Accordingly, it was confirmed that there was no change in the amount of the gas-phase $CF_4$ in the condenser.

Subsequently, a control group and one of the strain of *B. saitens* of Example 1 and the *E.coli* strain of Example 2 were each were each inoculated on an LB medium in the condenser using a syringe. Here, the control group included a wild-type strain of *Bacillus cereus.* In the LB medium on which the strains were inoculated, an initial concentration was 5.0 on the basis of $OD_{600}$. The LB culture had a circulation rate of about 4 mL/min, and the temperature inside the condenser was maintained at 30°C□. Following the inoculation and after the elapse of 42, 90, and 140 hours, the amount of the gas-phase $CF_4$ in the condenser was confirmed by GC-MS. Here, the decomposition rate of the gas-phase $CF_4$ was calculated according to Equation 1, and the results are shown in FIGS. 3 and 4.

<Equation 1>

**Decomposition rate of $CF_4$ = [(Initial amount of $CF_4$ – amount of $CF_4$ after reaction) / initial amount of $CF_4$] × 100**

FIG. 3 is a graph showing decomposition rates of $CF_4$ when a strain of *B. saitens* was brought into contact with the fluorine-containing compound while being subjected to circulation in a glass Dimroth coil reflux condenser.

FIG. 4 is a graph showing decomposition rates of $CF_4$ when a strain of *E. coli* BL21/pET-BS01766 was brought into contact with a fluorine-containing compound while being subjected to circulation in a glass Dimroth coil reflux condenser.

FIG. 5 is a graph showing decomposition rates of $CF_4$ when a strain of *Bacillus cereus* was brought into contact with a fluorine-containing compound while being subjected to circulation in a glass Dimroth coil reflux condenser.

As shown in FIGS. 3 to 5, the strain of *B. saitens* and the strain of *E. coli* BL21/pET-BS01766 showed significantly high decomposition rates, compared to the decomposition rate of the control group.

FIG. 6 is a schematic diagram for decomposing $CF_4$ by applying a gas-phase circulation process using a microorganism.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Moreover, any combination of the above-described elements in all possible variations thereof is disclosed herewith unless otherwise indicated herein or otherwise clearly contradicted by context.

## Claims

**1.** A microorganism deposited with the Korean Collection for Type Culture (KCTC) under accession no. 13219BP and referred to as *Bacillus saitens*, which microorganism when contacted with a sample containing a fluorine-containing compound of Formula 1 or 2 reduces the concentration of the fluorine containing compound in the sample:

<Formula 1> $C(R_1)(R_2)(R_3)(R_4)$

<Formula 2> $(R_5)(R_6)(R_7)C\text{-}[C(R_{11})(R_{12})]n\text{-}C(R_8)(R_9)(R_{10})$

wherein, in Formulae 1 and 2,

n is an integer from 0 to 10, ;
$R_1$, $R_2$, $R_3$, and $R_4$ are each independently fluorine (F), chlorine (Cl), bromine (Br), iodine (I), or hydrogen (H), wherein at least one of $R_1$, $R_2$, $R_3$, or $R_4$ is F; and
$R_5$, $R_6$, $R_7$, $R_8$, $R_9$, $R_{10}$, $R_{11}$, and $R_{12}$ are each independently F, Cl, Br, I, or H, wherein at least one of $R_5$, $R_6$, $R_7$, $R_8$, $R_9$, $R_{10}$, $R_{11}$, or $R_{12}$ is F;
and wherein, when n is equal or larger than 2, each $R_{11}$ is identical to or different from each other, and each $R_{12}$ is identical to or different from each other.

**2.** A microorganism comprising a genetic modification that increases the level of a polypeptide having a sequence identity of 90% or more with respect to an amino acid sequence of SEQ ID NO: 1, 3, or 5.

**3.** The microorganism of claim 2, wherein the genetic modification is an increase in copy number of a gene encoding the polypeptide, and/or wherein the microorganism comprises an exogenous gene encoding the polypeptide.

**4.** The recombinant microorganism of claim 3, wherein the gene has a sequence identity of 90% or more with respect to a nucleotide sequence of SEQ ID NO: 2, 4, or 6.

**5.** The recombinant microorganism of any one of claims 2 to 4, wherein the recombinant microorganism belongs to the genus *Escherichia, Bacillus, Pseudomonas, Xanthobacter,* or *Saccharomyces.*

**6.** A composition comprising

(a) *Bacillus saitens* (KCTC 13219BP); a microorganism according to any one of claims 2 to 5; or a combination thereof;
and
(b) a fluorine-containing compound of Formula 1 or Formula 2:

<Formula 1> $C(R_1)(R_2)(R_3)(R_4)$

<Formula 2> $(R_5)(R_6)(R_7)C\text{-}[C(R_n)(R_{12})]n\text{-}C(R_8)(R_9)(R_{10})$

wherein, in Formulae 1 and 2,

n is an integer from 0 to 10, ;
$R_1$, $R_2$, $R_3$, and $R_4$ are each independently fluorine (F), chlorine (Cl), bromine (Br), iodine (I), or hydrogen (H), wherein at least one of $R_1$, $R_2$, $R_3$, or $R_4$ is F; and
$R_5$, $R_6$, $R_7$, $R_8$, $R_9$, $R_{10}$, $R_{11}$, and $R_{12}$ are each independently F, Cl, Br, I, or H, wherein at least one of $R_5$, $R_6$, $R_7$, $R_8$, $R_9$, $R_{10}$, $R_{11}$, or $R_{12}$ is F;
and wherein, when n is equal or larger than 2, each $R_{11}$ is identical to or different from each other, and each $R_{12}$ is identical to or different from each other.

**7.** The composition of claim 6, wherein the fluorine-containing compound of Formula 1 or Formula 2 is in a liquid or gaseous state.

**8.** A method of reducing a concentration of a fluorine-containing compound in a sample, the method comprising:

contacting a sample comprising a fluorine-containing compound with *Bacillus saitens* (KCTC 13219BP) or a microorganism according to any one of claims 2 to 5, so as to reduce the concentration of the fluorine-containing compound in the sample,
wherein the fluorine-containing compound is represented by Formula 1 or Formula 2:

<Formula 1> $C(R_1)(R_2)(R_3)(R_4)$

<Formula 2> $(R_5)(R_6)(R_7)C\text{-}[C(R_{11})(R_{12})]n\text{-}C(R_8)(R_9)(R_{10})$

wherein, in Formulae 1 and 2,

n is an integer from 0 to 10, ;
$R_1$, $R_2$, $R_3$, and $R_4$ are each independently fluorine (F), chlorine (Cl), bromine (Br), iodine (I), or hydrogen (H), wherein at least one of $R_1$, $R_2$, $R_3$, or $R_4$ is F; and
$R_5$, $R_6$, $R_7$, $R_8$, $R_9$, $R_{10}$, $R_{11}$, and $R_{12}$ are each independently F, Cl, Br, I, or H, wherein at least one of $R_5$, $R_6$, $R_7$, $R_8$, $R_9$, $R_{10}$, $R_{11}$, or $R_{12}$ is F;
and wherein, when n is equal or larger than 2, each $R_{11}$ is identical to or different from each other, and each $R_{12}$ is identical to or different from each other.

**9.** The method of claim 8, wherein the contacting (a) is performed in an air-tight sealed container, (b) comprises culturing or incubating *B. saitens* (KCTC 13219BP) or the microorganism while in contact with the sample, and/or (c) comprises culturing *B. saitens* (KCTC 13219BP) or the microorganism under conditions in which *B. saitens* (KCTC 13219BP) or the microorganism proliferates in an air-tight sealed container.

**10.** The method of claim 8, wherein the contacting comprises, in an exhaust gas decomposition device comprising one or more reactors each of which comprises at least one first inlet and a first outlet:

injecting the sample into the exhaust gas decomposition device; and
injecting *B. saitens* (KCTC 13219BP) or the microorganism through the at least one first inlet so that *B. saitens* (KCTC 13219BP) or the microorganism contacts the sample and the resulting mixture is discharged through the first outlet.

**11.** The method of claim 10, wherein the exhaust gas decomposition device comprises a second inlet and a second outlet, the sample is injected through the second inlet and discharged through the second outlet, and a direction in which B. *saitens* (KCTC 13219BP) or the microorganism moves is opposite to a direction in which the sample moves, and/or wherein a fluid thin film comprising *B. saitens* (KCTC 13219BP) or the microorganism is formed on an inner wall of the one or more reactors, or on a packing material when the one or more reactors comprises a packing material.

**12.** A polypeptide having a sequence identity of 90% or more with respect to an amino acid sequence of SEQ ID NO: 1, 3, or 5.

**13.** A polynucleotide encoding a polypeptide comprising the amino acid sequence of SEQ ID NO: 1, 3, or 5 or an amino acid sequence with at least 90% sequence identity thereto, preferably a polynucleotide having nucleotide sequence of SEQ ID NO: 2, 4, or 6, or a vector comprising a promoter operably linked to a nucleic acid sequence.

**14.** A method of preparing a recombinant microorganism of claim 3, the method comprising introducing into a microorganism an exogenous, optionally heterologous, polynucleotide encoding a polypeptide comprising the amino acid sequence of SEQ ID NO: 1, 3, or 5 or an amino acid sequence with at least 90% sequence identity thereto.

**15.** The method of claim 14, wherein the polynucleotide comprises a nucleic acid sequence of SEQ ID NO: 2, 4, or 6.

FIG. 1

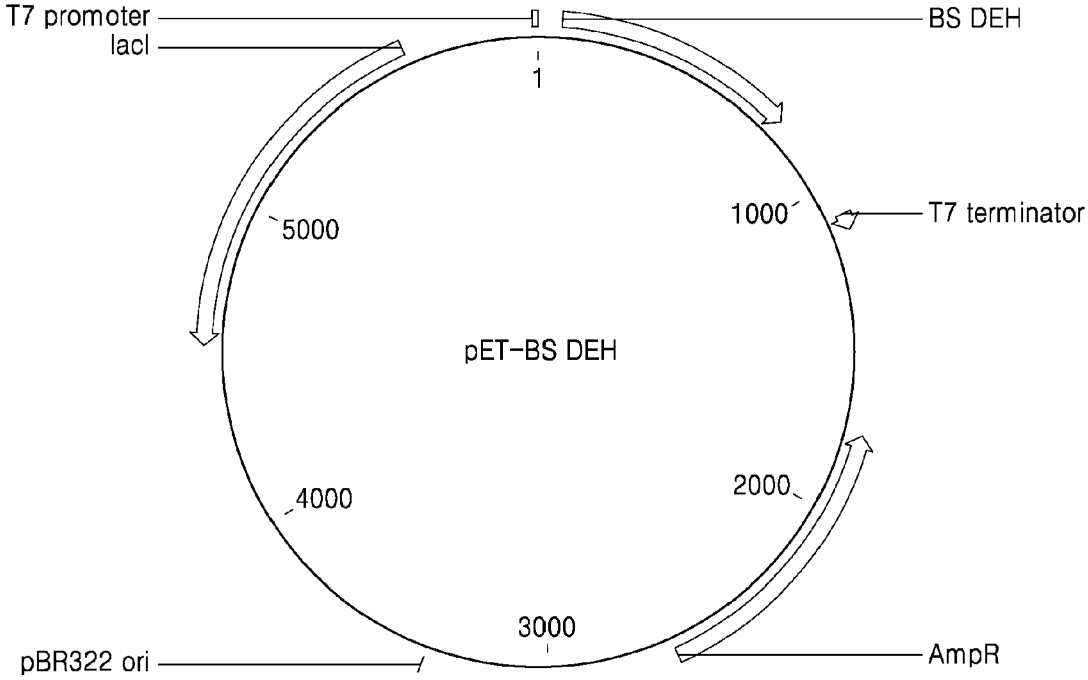


*BS DEH: GENE_01070, GENE_01766, OR GENE_03901

FIG. 2

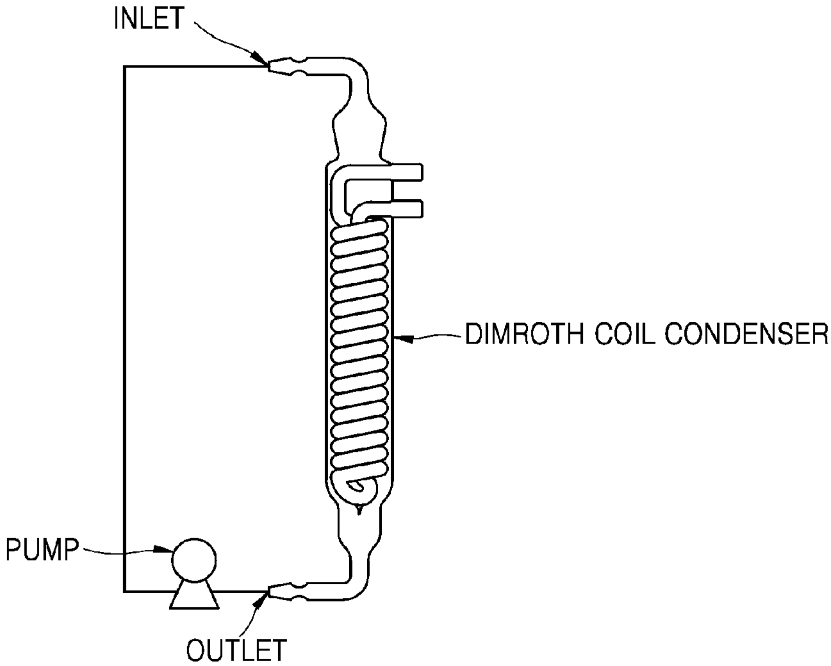

FIG. 3

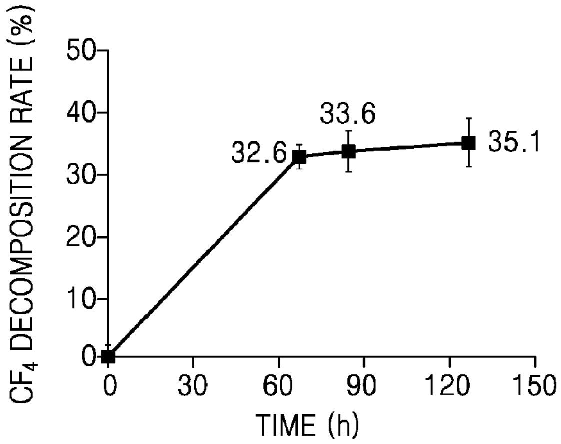

$CF_4$ DECOMPOSITION RATE (%)
50
40
30
20
10
0
0
30
60
90
120
150
TIME (h)
32.6
33.6
35.1


FIG. 4

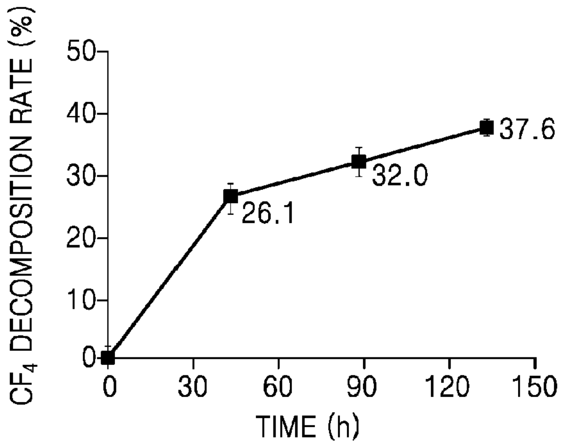

$CF_4$ DECOMPOSITION RATE (%)
50
40
30
20
10
0
0
30
60
90
120
150
TIME (h)
26.1
32.0
37.6

FIG. 5

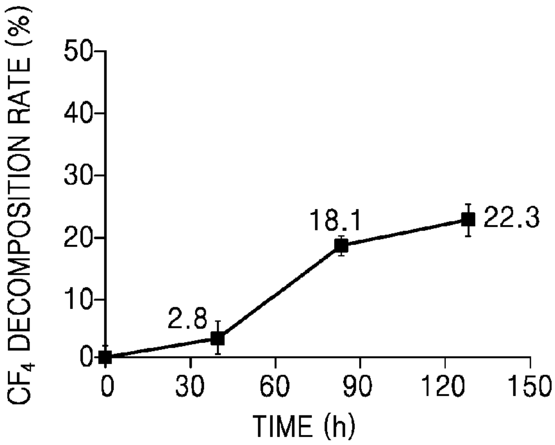
CF4 DECOMPOSITION RATE (%)
50
40
30
20
10
0
0
30
60
90
120
150
TIME (h)
2.8
18.1
22.3

FIG. 6

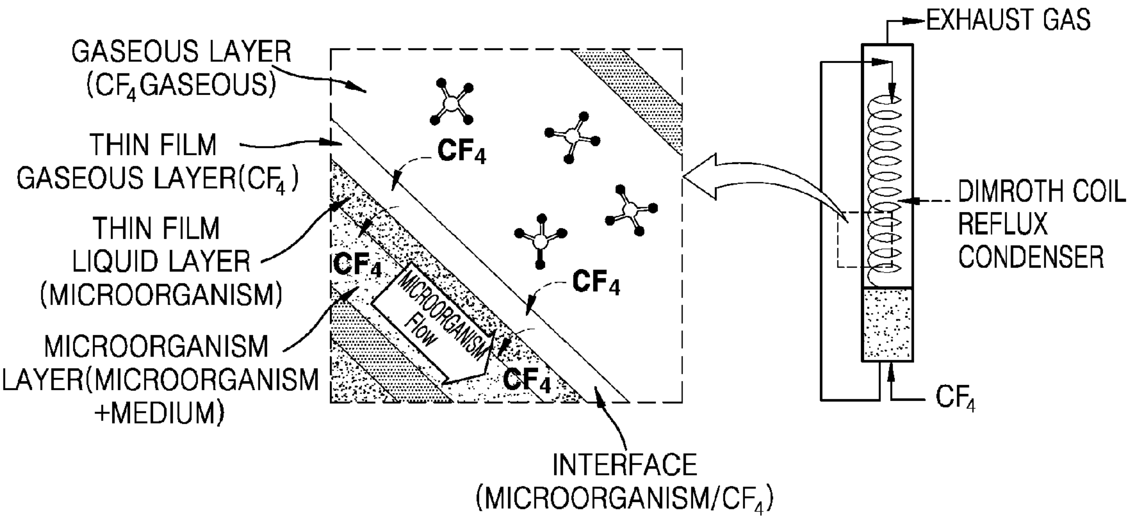
GASEOUS LAYER
(CF4GASEOUS)
THIN FILM
GASEOUS LAYER(CF4)
THIN FILM
LIQUID LAYER
(MICROORGANISM)
MICROORGANISM
LAYER(MICROORGANISM
+MEDIUM)
CF4
CF4
CF4
CF4
MICROORGANISM
Flow
INTERFACE
(MICROORGANISM/CF4)
EXHAUST GAS
DIMROTH COIL
REFLUX
CONDENSER
CF4

Europäisches Patentamt
European Patent Office
Office européen des brevets

# EUROPEAN SEARCH REPORT

Application Number
EP 18 18 5116

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X,P | EP 3 309 246 A1 (SAMSUNG ELECTRONICS CO LTD [KR]) 18 April 2018 (2018-04-18) * the whole document * * in particular claims 1-15 * ----- | 2-9, 12-15 | INV. C12R1/07 C12N9/14 B01D53/70 C02F3/34 |
| X | SHRABONI MUKHERJEE ET AL: "Characterization of a fluoride-resistant bacterium Acinetobacter sp. RH5 towards assessment of its water defluoridation capability", APPLIED WATER SCIENCE, vol. 7, no. 4, 18 December 2015 (2015-12-18), pages 1923-1930, XP055510027, ISSN: 2190-5487, DOI: 10.1007/s13201-015-0370-3 * the whole document * * in particular abstract * ----- | 1 | |
| X | ANOUK F DUQUE ET AL: "Isolation and characterization of astrain able to degrade 2-fluorophenol", APPLIED MICROBIOLOGY AND BIOTECHNOLOGY, SPRINGER, BERLIN, DE, vol. 95, no. 2, 19 November 2011 (2011-11-19), pages 511-520, XP035068538, ISSN: 1432-0614, DOI: 10.1007/S00253-011-3696-2 * the whole document * * in particular abstract * ----- | 1 | TECHNICAL FIELDS SEARCHED (IPC) C12R C12N B01D C02F |
| X | EP 3 093 337 A2 (SAMSUNG ELECTRONICS CO LTD [KR]) 16 November 2016 (2016-11-16) * the whole document * * in particular claims 1-15 * ----- -/-- | 2-15 | |

The present search report has been drawn up for all claims

2

| Place of search | Date of completion of the search | Examiner |
|---|---|---|
| Munich | 27 September 2018 | Dumont, Elisabeth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 18 18 5116

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/114348 A1 (CHU HUNSU [KR] ET AL) 27 April 2017 (2017-04-27)<br>* the whole document *<br>* in particular claims 1-20 *<br>----- | 2-15 | |
| A | US 2007/178578 A1 (CHALMER PAUL D [US] ET AL) 2 August 2007 (2007-08-02)<br>* the whole document *<br>* in particular par. 4, 10, 26-28; Fig. 4 *<br>----- | 10,11 | |
| X,P | EP 3 329 981 A2 (SAMSUNG ELECTRONICS CO LTD [KR]) 6 June 2018 (2018-06-06)<br>* the whole document *<br>* in particular claims 1-15 *<br>----- | 1-15 | |
| A,P | Aparna Singh ET AL: "A reappraisal on biodegradation of fluoride compounds: role of microbes",<br>Water and Environment Journal . December 2017,<br>22 December 2017 (2017-12-22), pages 1-7, XP055510036,<br>Retrieved from the Internet:<br>URL:https://onlinelibrary.wiley.com/doi/pdf/10.1111/wej.12322<br>[retrieved on 2018-09-26]<br>* the whole document *<br>* in particular Table 2 *<br>----- | 1-15 | TECHNICAL FIELDS SEARCHED (IPC) |

The present search report has been drawn up for all claims

| Place of search | Date of completion of the search | Examiner |
|---|---|---|
| Munich | 27 September 2018 | Dumont, Elisabeth |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

## ANNEX TO THE EUROPEAN SEARCH REPORT ON EUROPEAN PATENT APPLICATION NO. EP 18 18 5116

This annex lists the patent family members relating to the patent documents cited in the above-mentioned European search report.
The members are as contained in the European Patent Office EDP file on
The European Patent Office is in no way liable for these particulars which are merely given for the purpose of information.

27-09-2018

| Patent document cited in search report | Publication date | Patent family member(s) | Publication date |
|---|---|---|---|
| EP 3309246 A1 | 18-04-2018 | CN 107955801 A | 24-04-2018 |
| | | EP 3309246 A1 | 18-04-2018 |
| | | KR 20180042016 A | 25-04-2018 |
| | | US 2018104647 A1 | 19-04-2018 |
| EP 3093337 A2 | 16-11-2016 | CN 106139887 A | 23-11-2016 |
| | | EP 3093337 A2 | 16-11-2016 |
| | | US 2016333359 A1 | 17-11-2016 |
| US 2017114348 A1 | 27-04-2017 | NONE | |
| US 2007178578 A1 | 02-08-2007 | NONE | |
| EP 3329981 A2 | 06-06-2018 | CN 108144434 A | 12-06-2018 |
| | | EP 3329981 A2 | 06-06-2018 |
| | | KR 20180063752 A | 12-06-2018 |
| | | US 2018154308 A1 | 07-06-2018 |

EPO FORM P0459

For more details about this annex : see Official Journal of the European Patent Office, No. 12/82